# DEMANDE DE BREVET EUROPEEN

(11) **EP 0 792 590 A1**
(43) Date de publication de la demande: **03.09.1997**
(21) Numéro de dépôt: 97400448.3
(22) Date de dépôt: 27.02.1997
(51) Int. Cl.: A23L 3/3571, A23L 3/3562, A61K 7/00

(54) **Composition alimentaire aqueuse essentiellement non lactée de qualité bactériologique constante et procédé pour inhiber la croissance bactérienne d'une composition alimentaire**

(30) Priorité: 29.02.1996 FR 9602572
(71) Demandeur: COMPAGNIE GERVAIS DANONE, 92300 Levallois Perret (FR)
(72) Inventeur: Colomban, François, 75014 Paris (FR); Fuhrmann, Benoît, 78690 Les Essarts Le Roi (FR); Modamio, Xavier, 91370 Verrieres Les Buissons (FR)
(74) Mandataire: Warcoin, Jacques

(57) **Abrégé**

La présente invention concerne une composition alimentaire aqueuse essentiellement non lactée de qualité bactériologique constante pendant une durée appropriée comprenant à titre de composant inhibant substantiellement le développement des bactéries, une quantité efficace d'au moins un monosaccharide permettant d'augmenter la pression osmotique du milieu et au moins une enzyme bactéricide.

Elle concerne également un procédé pour inhiber la croissance bactérienne ou pour diminuer sensiblement la population bactérienne d'une composition alimentaire ou cosmétique, caractérisé en ce qu'on ajoute à ladite composition une quantité efficace d'un monosaccharide permettant d'augmenter la pression osmotique du milieu et au moins une enzyme bactéricide.

## Description

La présente invention concerne une composition alimentaire aqueuse essentiellement non lactée de qualité bactériologique constante pendant une durée appropriée.

Elle a également pour objet un procédé pour inhiber la croissance bactérienne ou pour diminuer sensiblement la population bactérienne d'une composition alimentaire ou cosmétique.

La présente invention se rapporte plus particulièrement aux compositions alimentaires à base de produits frais devant être conservés à basse température par exemple au réfrigérateur, pendant une durée appropriée, compte tenu de la chaîne de distribution et de l'utilisation plus ou moins différée desdits produits par le consommateur.

Lors de la fabrication de compositions alimentaires, notamment à base de produits frais, il peut être nécessaire d'y incorporer certains ingrédients et aliments (chocolat, jaune d'oeuf, poudres diverses, céréales, épices, biscuits, fruits secs, fruits déshydratés ...) d'intérêt nutritionnel, organoleptique ou technologique qui peuvent en altérer les qualités marchandes et hygiéniques. Par exemple, l'adjonction de chocolat dans la composition de desserts lactés peut entraîner une contamination microbienne du produit fini due notamment à la présence de bactéries sporulées dans le chocolat.

La recherche de solutions susceptibles de supprimer ces inconvénients est d'un intérêt majeur dans l'utilisation de ces ingrédients notamment pour la préparation de produits nouveaux.

A ce jour, les technologies classiques de traitement thermique anti-microbien sont susceptibles de modifier les qualités organoleptiques, nutritionnelles ou technologiques des aliments et ingrédients traités. De plus, le type de microorganismes présents, notamment les bactéries sporulées, et l'environnement dans lequel elles se trouvent, par exemple les ingrédients à faible activité de l'eau, voire à teneur en lipides ou en protéines élevée, entraînent des conditions de traitement thermique peu compatibles avec les réalités industrielles.

Ces dernières années, de nouveaux procédés technologiques ont été étudiés tels que les très hautes pressions, l'ionisation, l'infrarouge, l'ultraviolet... Cependant, ces procédés trouvent également leur limite, le compromis entre l'efficacité anti-microbienne et le maintien des qualités organoleptiques n'étant pas toujours satisfaisant.

En règle générale, les produits frais doivent conserver leur qualité organoleptique et leurs caractéristiques bactériologiques pendant 28 jours à 6°C. Cependant ces produits peuvent subir des variations thermiques lors de rupture(s) de la chaîne du froid, de ce fait il est préférable de maintenir leur stabilité pendant au moins 3 heures de conservation à 30°C et bien sur 28 jours de conservation à 10°C.

L'objet de la présente invention est de proposer de nouveaux moyens et des compositions alimentaires utilisant lesdits moyens permettant de répondre en partie ou totalement aux critères indiqués ci-après :
- maintien de l'intérêt organoleptique, nutritionnel ou technologique de l'ingrédient traité par le procédé.
- réduction satisfaisante de la flore microbienne notamment des bactéries pathogènes *(Bacillus cereus, Escherichia coli, Staphylocoque aureus, Salmonella enteritidis, Listeria monocytogenes...*) compte tenu des critères bactériologiques exigés pour la conservation des produits finis pendant toute leur durée d'utilisation normale.
- compatibilité alimentaire du procédé de traitement et des éléments pouvant entrer dans ce traitement.
- coût de traitement compatible avec la valeur des produits finis.
- traitement pouvant être industrialisé.

La présente invention concerne en premier lieu une composition alimentaire aqueuse essentiellement non lactée de qualité bactériologique constante pendant une durée appropriée comprenant à titre de composant inhibant substantiellement le développement des bactéries, une quantité efficace d'au moins un monosaccharide permettant d'augmenter la pression osmotique du milieu et au moins une enzyme bactéricide.

Par l'expression "essentiellement non lactée", on entend que la composition ne comprend ni de protéine ni de corps gras du lait. Par contre, la composition alimentaire pourra éventuellement comporter du lactosérum.

Par l'expression "qualité bactériologique constante pendant une durée appropriée", on entend notamment les qualités déjà indiquées ci-dessus, notamment en ce qui concerne la possibilité pour les compositions selon l'invention de voir les populations bactérienne être stabilisées voire diminuées au cours d'une conservation de plusieurs jours à 4°C, 6°C ou 10°C.

Par l'expression "quantité efficace", on entend une quantité suffisante pour permettre de répondre aux critères de conservation indiqués ci-dessus.

L'invention concerne également un procédé pour inhiber la croissance bactérienne ou pour diminuer sensiblement la population bactérienne d'une composition alimentaire ou éventuellement cosmétique, caractérisé en ce que l'on ajoute à ladite composition une quantité efficace d'un monosaccharide permettant d'augmenter la pression osmotique du milieu et au moins une enzyme bactéricide.

La demanderesse a constaté en effet de façon inattendue que la combinaison des deux moyens :
augmentation de la pression osmotique obtenue par un monosaccharide
et présence d'une enzyme bactéricide
conduisait à des compositions pouvant être conservées selon les critères exigés.

Un moyen pour évaluer la pression osmotique d'une composition consiste à mesurer l'activité de l'eau.

L'activité de l'eau d'un produit est une notion qui est bien connue dans le domaine alimentaire, cette mesure souvent abrégée sous forme Aw mesure la disponibilité de l'eau dans un échantillon. Dans la plupart des cas, cette activité de l'eau n'est pas proportionnelle à la teneur en eau du produit.

A titre d'exemple, on peut mentionner :
- chocolat : environ 1 % d'eau : Aw = 0,50
- glycérol à 10 % dans l'eau : Aw = 0,95

Les méthodes permettant la mesure de l'activité de l'eau d'un produit sont connues de l'homme du métier.

Dans le cas d'une composition alimentaire aqueuse essentiellement non lactée, l'activité de l'eau, sans présence du composant inhibant substantiellement le développement des bactéries, est d'environ 0,99.

Selon un aspect de la présente invention, la composition alimentaire est caractérisée en ce qu'elle présente avant l'ajout du composant une activité de l'eau supérieure à 0,96 et en ce que le monosaccharide permet d'abaisser l'activité de l'eau en dessous de 0,95, de préférence en dessous de 0,94.

En général, l'activité de l'eau de la composition alimentaire résultante sera donc comprise entre 0,90 et 0,95 ou avantageusement entre 0,90 et 0,94.

Selon un autre aspect de la présente invention, la composition alimentaire comprend un ou plusieurs aliments susceptibles d'entraîner une contamination bactérienne de ladite composition alimentaire.

Parmi ces aliments, on peut citer ceux choisis dans le groupe constitué par le chocolat, le jaune d'oeuf, les céréales, les épices, les biscuits, les fruits secs, les fruits déshydratés et les poudres à base de ces produits. Le blanc d'oeuf peut également s'avérer dans certaines conditions légèrement contaminant.

La composition alimentaire peut comprendre également divers autres agents tels que des agents épaisissants, texturants ou gélifiants. Parmi ceux-ci, on peut citer la gomme xanthane, l'amidon ou d'autres agents équivalents.

Les monosaccharides permettant de mettre en oeuvre la présente invention sont de préférence choisis dans le groupe constitué par le glucose, le D-glucose ou dextrose, le fructose.

Bien entendu, l'invention n'est pas limitée aux monosaccharides spécifiquement indiqués, mais s'étend au contraire aux autres monosaccharides qui permettraient également d'abaisser la pression osmotique et plus précisément l'activité de l'eau, comme indiqué ci-dessus.

Avantageusement, la composition alimentaire comprendra en pourcentage en poids de 6 à 25 % desdits monosaccharides, avantageusement 10 à 20%.

Les enzymes convenant particulièrement sont choisies dans le groupe de celles contenues dans les blancs d'oeufs, notamment l'oeuf des poules dont on peut citer l'ovotransférine ou conalbumine, l'avidine, le lysozyme.

On sait que ces enzymes sont présentes dans le blanc d'oeuf dans des quantités voisines de 12 % pour l'ovotransférine, 3,5 % pour le lysozyme et 0,05 % pour l'avidine.

Une composition alimentaire préférée comprendra au moins du lysozyme. On sait que le lysozyme dissout la paroi de certaines bactéries en hydrolysant la substance mucocomplexe responsable de la structure rigide de la cellule. Cette enzyme est plus active sur les bactéries gram+ que sur les bactéries gram-.

De préférence, la composition alimentaire comprendra au moins 100 ppm de lysozyme et avantageusement entre 300 et 3000 ppm. La limite supérieure n'est pas cruciale, car des quantités supérieures pourraient être utilisées bien que cela ne procure aucun effet particulier.
Une variante préférée consiste à utiliser du blanc d'oeuf, les enzymes bactéricides, notamment le lysozyme étant contenu dans ledit blanc d'oeuf.

L'invention est particulièrement intéressante pour les compositions alimentaires présentant un pH voisin de la neutralité et de façon générale compris entre 5,5 et 9. On rappellera en effet que les compositions alimentaires de pH inférieur à 4,5 sont par nature bactériostatiques voire bactéricides.

De préférence, le pH de ces compositions est compris entre 6 et 7.

Les compositions alimentaires selon l'invention avantageusement sont caractérisées en ce qu'elles sont de qualité bactériologique constante pendant au moins 28 jours à 10°C.

Une seconde caractéristique avantageuse de ces compositions est qu'elle présente une qualité bactériologique constante pendant au moins 6 heures à 30°C.

Une caractéristique importante de certaines des compositions selon l'invention est qu'il s'agit d'une composition non stérile qui, par les moyens décrits précédemment, ne présente aucun risque de dégradation bactériologique pendant la durée requise.

Une variante préférée selon l'invention concerne une composition alimentaire répondant aux critères de qualité bactériologique indiqués ci-dessus, caractérisée en ce qu'elle est est sous la forme d'un produit frais, notamment mousseux, ayant une activité de l'eau comprise entre 0,9 et 0,95, de pH compris entre 5,5 et 9, et présentant la composition suivante en pourcentage en poids :
- aliment susceptible d'entraîner une contamination bactérienne tel que le chocolat 20 à 40 %
- jaune d'oeuf 5 à 15 %
- monosaccharide 10 à 20 %
- blanc d'oeuf 20 à 45 %
- eau 5 à 17 %
et éventuellement de l'amidon, du sirop de glucose, une gomme arabique, du NaCl.

De préférence, cette composition est caractérisée en ce qu'elle présente la composition suivante en pourcentage en poids :
- chocolat 21 à 35 %
- jaune d'oeuf 5 à 15 %
- dextrose 10 à 20 %
- blanc d'oeuf 25 à 40 %
- eau 5 à 17 %
et éventuellement de l'amidon, du sirop de glucose, une gomme arabique, du NaCl.

Cette compositions alimentaire peut être notamment un dessert.

On a déjà expliqué dans la descriptioin ci-dessus que l'invention était relative à une composition alimentaire comportant un composant permettant d'augmenter la pression osmotique du milieu (ce qui est mesuré expérimentalement par un abaissement de l'activité de l'eau) au moyen d'un monosaccharide, notamment le dextrose, et par la présence d'une enzyme bactéricide.

L'invention concerne en conséquence également un procédé pour inhiber la croissance bactérienne ou pour diminuer sensiblement la population bactérienne d'une composition alimentaire ou cosmétique, caractérisé en ce qu'on ajoute à ladite composition une quantité efficace d'un monosaccharide permettant d'augmenter la pression osmotique du milieu et au moins une enzyme bactéricide comme précédemment.

Ce procédé bien qu'applicable en priorité aux compositions alimentaires peut également être appliqué aux compositions cosmétiques requérant une stabilité bactériologique.

Par compositions cosmétiques, on entend toute composition devant être appliquée de façon topique externe.

Les bactéries concernées par ce type de procédé sont nombreuses et disperses et sont notamment les bactéries pathogènes, les bactéries sporulées et les bactéries contaminantes.

On peut citer à titre non limitatif, les bactéries suivantes :

*Staphylococcus hominis*, *Staphylococcus warnerie, Staphylococcus aureus*, *Listeria monocytogenes, Salmonella typhimurium, Salmonella enteritidis*, 2 souches *Salmonella* isolées d'ovoproduits, 2 souches *Salmonella* isolées de fèves de cacao, *Bacillus* cereus, *Bacillus subtilis, Bacillus pumilus, micrococcus spp., Enterococcus faecium*, *Enterobacter cloacae, Escherichia coli*, *Klebsiella pneumoniae*, *Leuconostoc spp*., *Pseudomonas aeruginosa, Flora Danica*.

Ces souches incluent les bactéries pathogènes d'incidence dans le domaine laitier et des ovoproduits ainsi que les bactéries les plus répandues dans le domaine laitier, chocolatier et d'ovoproduits.

Les exemples ci-après illustrent la présente invention, sans toutefois la limiter.

### Exemple 1

### 1. Composition testée

L'exemple suivant a été réalisé sur une mousse au chocolat non traitée thermiquement de composition suivante en pourcentage en poids :
. chocolat 28 %
. jaune d'oeuf 10 %
. sirop de glucose 1,9 %
. amidon 0,56 %
. NaCl 0,056 %
. xanthane 0,019 %
. blanc d'oeuf (dont 800 ppm environ de lysozyme) 32,5 %
. dextrose 15,9 %
. eau q.s.p 100 %

L'activité de l'eau d'une telle composition est de 0,928 et le pH est de 6,5.

Cette mousse au chocolat est réalisée selon le procédé suivant :
a) Mélange et stérilisation des ingrédients (amidon, NaCI, xanthane, sirop de glucose, dextrose, eau)
b) Injection de blanc d'oeuf
c) Foisonnement
d) Injection jaune d'oeuf
e) Injection chocolat
f) Mélange

On remarquera qu'au delà de la séquence b), aucun traitement thermique ou d'assainissement bactérien classique n'est appliqué.

### 2. Essais de contamination

On a contaminé artificiellement cette mousse par différents types de bactéries potentiellement pathogènes (*B*. *cereus , E. coli, S*. *aureus*, *S*. *enteritidis*, *L. monocytogenes*). L'évolution de cette flore a été étudiée à 1, 7, 14, 21 et 28 jours de conservation à 10°C. Les résultats représentés dans le tableau ci-dessous mettent en évidence une absence de croissance de la souche de B. cereus voire une réduction significative des souches de *E. coli, de S. aureus, de S. enteritidis et de L. monocytogenes.*

| Bactéries | Numération en UFC/g après différents jours (J) de conservation à 10°C | | | | | |
|---|---|---|---|---|---|---|
| | J0 | J1 | J7 | J14 | J21 | J28 |
| *B. cereus* | 8.5 10³ | 3.0 10³ | 4.0 10³ | 4.0 10³ | 1.0 10⁴ | 2.2 10³ |
| *E. coli* | 1.3 10⁶ | 1.2 10⁶ | 6.5 10⁵ | 1.8 10⁵ | 4.510⁴ | 1.7 10⁴ |
| *S. aureus* | 4.0 10⁶ | 9.5 10⁵ | 3.0 10⁴ | 3.0 10³ | 1.010² | 50 |
| *S. enteritidis* | 1.8 10⁴ | 1.2 10⁴ | 8 10³ | 4.1 10³ | 1.310³ | 4.9 1O² |
| *L. monocytogenes* | 1.8 10⁴ | 9.3 10³ | 1.3 10³ | 3.3 10² | 1.41O² | 1.31O² |

Après 24 heures de conservation à 30°C, il n'a pas été constaté de croissance microbienne dans la mousse au chocolat selon les résultats indiqués ci-dessous :

| Bactéries | Numération en UFC/g après 24 heures (H24) de conservation à 30°C | |
|---|---|---|
| | H 0 | H 24 |
| *B. cereus* | 1.3 10⁴ | 6.5 10³ |
| *E. coli* | 3.0 10⁴ | 8.0 10³ |
| *S. aureus* | 8.0 10⁴ | 2.5 10³ |

Par un choix judicieux de la pression osmotique et de l'activité antibactérienne de composants du blanc d'oeuf, les qualités bactériologiques d'une mousse au chocolat ont pu être maintenues, tout en conservant ses caractéristiques organoleptiques.

### Exemple 2 - Essai in vitro sur l'influence du pourcentage de dextrose et de lysozyme (LYS en ppm) sur la croissance d'une souche de Staphylocoque aureus.

La numération en log (UFC/g) a été mesurée en fonction du temps de conservation à 10°C en jours pour cinq essais faisant intervenir des concentrations différentes de dextrose et de lysozyme.

| Essai | Dex (%) | LYS (ppm)* |
|---|---|---|
| 1 | 0 | 0 |
| 2 | 0 | 1000 |
| 3 | 24,2 | 500 |
| 4 | 41,7 | 0 |
| 5 | 41,7 | 1000 |

| | | |
|---|---|---|
| * La quantité de lysozyme est déterminée par une concentration en chlorhydrate de lysozyme. | | |

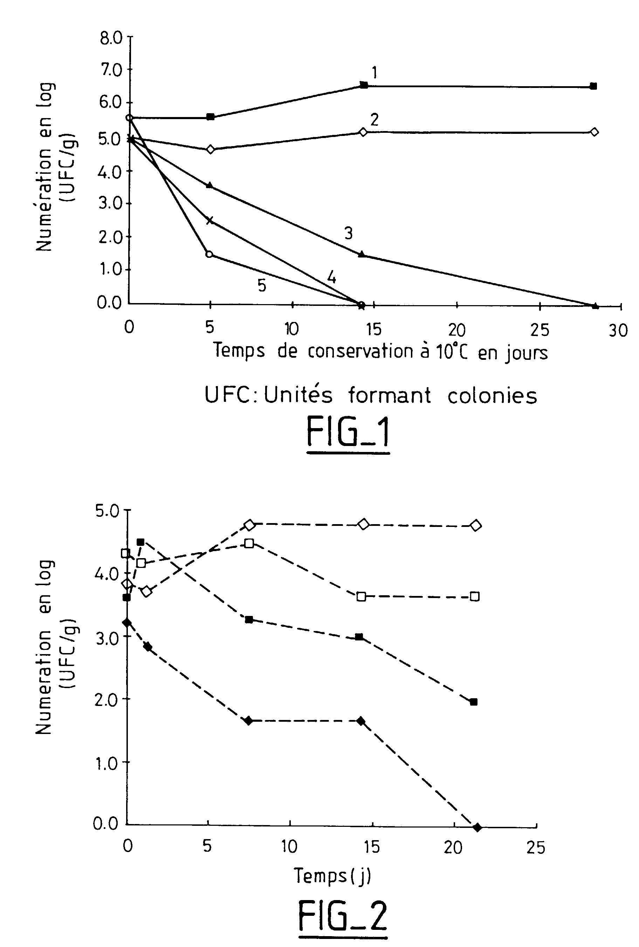

Les résultats sont indiqués à la figure 1 et démontrent l'effet avantageux du composant selon l'invention.

### Exemple 3 - Essai comparatif

Une mousse au chocolat similaire à celle de l'exemple 1 mais comportant la composition en sucre ci-dessous a été testée dans les mêmes conditions que dans l'exemple 1 après une contamination par *E. coli* et S. *aureus.*

| Composition (comparative) | |
|---|---|
| Blanc d'oeuf | 30 % |
| Chocolat | 27 % |
| Beurre | 20 % |
| Saccharose | 12 % |
| Jaune d'oeuf | 8 % |
| Poudre de blanc d'oeuf Aw = 0,93. | 3 % |

Après avoir contaminé artificiellement les compositions des exemples 1 et 3 par une souche de S. aureus et une souche de *E. coli,* l'évolution de la flore a été étudiée après 1, 7, 14, 21 et 28 jours de conservation à 10°C. Les résultats présentés dans la figure 2 annexée mettent en évidence une réduction significative des souches de *E. coli* et de *S. aureus* dans la composition de l'exemple 1, alors que pour la composition comparative, on observe une stabilité de la population bactérienne

### Explication des légendes de la figure 2

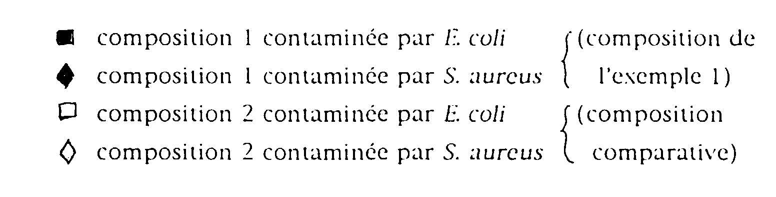

## Revendications

1. Composition alimentaire aqueuse essentiellement non lactée de qualité bactériologique constante pendant une dunée appropriée comprenant à titre de composant inhibant substantiellement le développement des bactéries, une quantité efficace d'au moins un monosaccharide permettant d'augmenter la pression osmotique du milieu et d'au moins une enzyme bactéricide.

2. Composition alimentaire selon la revendication 1, caractérisée en ce qu'elle comprend un ou plusieurs aliments susceptibles d'entraîner une contamination bactérienne de ladite composition alimentaire.

3. Composition alimentaire selon la revendication 2, caractérisée en ce que les aliments susceptibles d'entraîner une contamination microbienne sont choisis dans le groupe constitué par le chocolat, le jaune d'oeuf, les céréales, les épices, les biscuits, les fruits secs, les fruits déshydratés, les poudres.

4. Composition alimentaire selon la revendication 1, caractérisée en ce qu'elle présente avant l'ajout du composant inhibant substantiellement le développement des bactéries, une activité de l'eau supérieure à 0,96 et en ce que le monosaccharide permet d'abaisser l'activité de l'eau en dessous de 0,95, de préférence en dessous de 0,94.

5. Composition alimentaire selon la revendication 1 ou 4, caractérisée en ce que le monosaccharide est choisi dans le groupe constitué par le glucose, le D-glucose ou dextrose, le fructose.

6. Composition alimentaire selon la revendication 1 ou 5, caractérisée en ce qu'elle comprend en pourcentage en poids 6 à 25 % de monosaccharide.

7. Composition alimentaire selon la revendication 6, caractérisée en ce qu'elle comprend en pourcentage en poids 10 à 20 % de monosaccharide.

8. Composition alimentaire selon la revendication 1, caractérisée en ce que l'enzyme bactéricide est choisie dans le groupe constitué par l'ovotransférine ou conalbumine, l'avidine, le lysozyme.

9. Composition alimentaire selon la revendication 8, caractérisée en ce qu'elle comprend entre 300 et 3000 ppm de lysozyme.

10. Composition alimentaire selon la revendication 1, 8 ou 9 caractérisée en ce qu'elle comprend du blanc d'oeuf et que les enzymes bactéricides sont contenues dans le blanc d'oeuf.

11. Composition alimentaire selon la revendication 1, caractérisée en ce que le pH est compris entre 5,5 et 9.

12. Composition alimentaire selon la revendication 1, caractérisée en ce qu'elle est de qualité bactériologique constante pendant au moins 28 jours à 10°C.

13. Composition alimentaire selon la revendication 1 ou 12, caractérisée en ce qu'elle est de qualité bactériologique constante pendant au moins 6 heures à 30°C.

14. Composition alimentaire selon la revendication 12 ou 13, caractérisée en ce qu'elle est sous la forme d'un produit frais, notamment mousseux, ayant une activité de l'eau comprise entre 0,9 et 0,95, de pH compris entre 5,5 et 9, et présentant la composition suivante en pourcentage en poids:
- aliment susceptible d'entraîner une contamination bactérienne tel que le chocolat 20 à 40 %
- jaune d'oeuf 5 à 15 %
- monosaccharide 10 à 20%
- blanc d'oeuf 20 à 45 %
- eau 5 à 17 %
et éventuellement de l'amidon, du sirop de glucose, une gomme arabique, du NaCI.

15. Composition alimentaire selon la revendication 14, notamment un dessert, caractérisée en ce qu'elle présente la composition suivante en pourcentage en poids :
- chocolat 23 à 35 %
- jaune d'oeuf 5 à 15 %
- dextrose 10 à 20 %
- blanc d'oeuf 25 à 40 %
- eau 5 à 17 %
et éventuellement de l'amidon, du sirop de glucose, une gomme arabique, du NaCl.

16. Procédé pour inhiber la croissance bactérienne ou pour diminuer sensiblement la population bactérienne d'une composition alimentaire ou cosmétique, caractérisé en ce qu'on ajoute à ladite composition une quantité efficace d'un monosaccharide permettant d'augmenter la pression osmotique du milieu et au moins une enzyme bactéricide comme définie aux revendications 1, 4 à 11.

17. Procédé selon la revendication 16, caractérisé en ce que les bactéries sont choisies dans le groupe constitué par les bactéries pathogènes, bactéries sporulées, bactéries contaminantes.
